# EUROPEAN PATENT SPECIFICATION

(11) **EP 4 189 300 B1**
(45) Date of publication and mention of the grant of the patent: **25.09.2024**
(21) Application number: 21733526.4
(22) Date of filing: 04.06.2021
(51) Int. Cl.: F24F 8/30, A61L 9/22

(54) **ION DIFFUSER AND CARTRIDGE FOR AN ION DIFFUSER**
IONENDIFFUSOR UND PATRONE FÜR EINEN IONEN DIFFUSOR
DIFFUSEUR DES IONS ET CARTOUCHE POUR UN DIFFUSEUR

(30) Priority: 27.07.2020 NL 2026143; 21.12.2020 GB 202020302
(43) Date of publication of application: 07.06.2023
(73) Proprietor: Real Scientists Limited, London EC4Y (GB)
(72) Inventor: KORNEV, Alexander, London EC4Y 0HP (GB); KOSTEVITCH, Siarhei, 4045 Limassol (CY); KOSTEVITCH, Sergei, 4045 Limassol (CY)
(74) Representative: Kramer, Dani
(86) International application number: PCT/GB2021/051400
(87) International publication number: WO 2022/023692

(56) References cited:
- EP-A1- 2 559 952
- WO-A1-2015/059336
- CN-A- 107 504 584
- US-A1- 2018 326 818

## Description

### Field of the invention

The present invention relates to an ion diffuser for generating ions as well as a capsule for use with the ion diffuser, a method of manufacturing the capsule and a method of operating the ion diffuser.

### Background to the Disclosure

In recent years, air ionisers have been developed which produce ions in order to purify air. In particular, ionisers have been developed that apply a large voltage to air in order to produce negative ions of Oxygen (O₂⁻) and Nitrogen (N₂⁻). These ions interact with particulate matter in the air in order to charge this particulate matter. The charged particulate matter can then be removed from the air by being attracted to a charged plate, or simply to nearby structures, such as walls.

However, such air ionisers require extremely large input powers and voltages in order to ionise the nitrogen or oxygen in the air. Furthermore, these air ionisers tend to produce ozone (O₃), which can be toxic for humans in large concentrations. Therefore, environments in which an air ioniser has been used may need to be cleaned before they can be used by humans.

US 2018/326818 A1 describes a method of controlling an air freshener device for a passenger compartment of a vehicle.

EP 2559952 A1 describes an air purifier which can effectively purify the air for a long period of time without increasing the capacity of an internal power supply and an external power supply.

### Summary of the invention

According to an aspect of the present invention, there is described an ion diffuser for generating ions, the ion diffuser comprising: a cavity arranged to receive a substance; the substance being located in the cavity; and a power source arranged to provide a current to the substance to cause the emission of ions from the substance, wherein the substance comprises an ionic gel, the ionic gel comprising an ionic liquid that is held by an inorganic or polymer matrix, as defined in claim 1.

Preferred embodiments of the invention are defined in the dependent claims.

Preferably, the ion diffuser comprising: a cavity arranged to receive a capsule; and a power source arranged to provide a current to the capsule to cause the emission of ions from the capsule.

Preferably, the ion diffuser (e.g. the cavity) is arranged to receive a plurality of different types of substances and/or capsules. Preferably, the ion diffuser is arranged to receive a plurality of capsules, where the capsules comprise different substances. Preferably, the ion diffuser is arranged to identify a type of the or each substance and/or capsule.

Preferably the ion diffuser is arranged to emit ions in a plasma state and/or the ion diffuser is arranged to emit a plasma. Preferably, the power source is arranged to provide the current so as to cause the emission of ions from the capsule in a plasma state.

Preferably, the ion diffuser is arranged to generate positive ions and/or both positive and negative ions.

Preferably, the substance comprises a material and/or element with an atomic number of greater than 8, and/or greater than 25, and/or greater than 50.

Preferably, the substance comprises a metal.

Preferably, the substance comprises a compound. Preferably, the power source comprises a battery.

Preferably, the power source is arranged to provide a power of no more than 20W, no more than 10W, no more than 5W, and/or no more than 4W.

Preferably, the power source comprises a transformer. Preferably, the transformer is arranged to provide a high voltage. Preferably, the transformer is arranged to provide a voltage of at least 10kV, at least 15kV, and/or at least 20kV.

Preferably, the ion diffuser comprises one or more electrodes connected to the power source. Preferably, the ion diffuser comprises an anode and/or a cathode.

Preferably, the ion diffuser is arranged to emit ions of an element.

Preferably, the ion diffuser is arranged to emit ions of a metal.

Preferably, the ion diffuser is arranged to receive a capsule comprising the substance. Preferably, the cavity is arranged to receive the capsule.

Preferably, the cavity is arranged to receive a capsule comprising one or more electrodes. Preferably, the cavity is arranged to receive a capsule comprising an anode and/or a cathode. Preferably, the power source is arranged to provide a current to an electrode of the capsule. Preferably, the power source is arranged to provide a spark to the substance and/or capsule. Preferably, the ion diffuser comprises a placement structure arranged to ensure a positioning of the substance and/or capsule in the cavity. Preferably, the placement structure comprises a magnet.

Preferably, the ion diffuser comprises a distribution mechanism arranged to distribute the ions. Preferably, the distribution mechanism comprises a fan.

Preferably, the ion diffuser comprises a charged plate.

Preferably, the ion diffuser comprises a capsule identifier arranged to identify a characteristic of the capsule. Preferably, the ion diffuser comprises one or more of: a capacity and/or volume of the substance and/or capsule; a type of the substance and/or capsule; a substance in the capsule; and an authorisation relating to the capsule.

Preferably, the current depends on the characteristic. Preferably, a duration and/or magnitude of the current depends on the characteristic.

Preferably, the ion diffuser comprises a filter arranged to selectively block the emission of matter from the ion diffuser.

Preferably, the ion diffuser comprises a processor and/or a user interface.

Preferably, the ion diffuser is arranged to provide one or more of: anions; and cations. Preferably, the ion diffuser comprises a filter arranged to prevent the emission of selected ions.

Preferably, the ion diffuser comprises a plurality of cavities arranged to receive a plurality of substances, wherein the ion diffuser is arranged to selectively provide a current to one or more of the substances. Preferably, the provision of current is based on a user input.

Preferably, the substance comprises one or more of: a metal; a halogen; a compound, preferably a compound comprising a metal and a halogen; a liquid acrylic polymer; and one or more of: platinum, silver, gold, magnesium, and potassium.

Preferably, the ion diffuser is a portable device.

Preferably, the ion diffuser is an ion diffuser for purifying air.

Preferably, the ion diffuser comprises an air purifying device.

Preferably, the ion diffuser comprises the capsule.

Preferably, the ion diffuser comprises a plurality of substances and/or capsules.

According to another aspect of the present invention, there is described a capsule for use with an ion diffuser as defined in claim 9.

Preferably, the capsule comprises one or more of: a metal; a halogen; and a compound.

Preferably, the capsule comprises a compound comprising a metal and a halogen.

Preferably, the capsule comprises a liquid acrylic polymer.

Preferably, the capsule comprises one or more of platinum, silver, gold, magnesium, and potassium.

Preferably, the capsule comprises one or more electrodes.

Preferably, the capsule is arranged to be refillable.

Preferably, the capsule comprises a power source. Preferably, the capsule comprises a rechargeable power source.

Preferably, the capsule comprises an identifier. Preferably, the identifier comprises a communication interface. Preferably, the identifier comprises a radio frequency identifier (RFID) interface and/or a near field communication (NFC) interface.

Preferably, the identifier is arranged to identify a characteristic of the capsule and/or a substance in the capsule.

Preferably, the capsule comprises a plurality of substance sections separated by a gap. Preferably, each of the substance sections comprises one or more of: a salt, an electrolyte, an ionic liquid, a porous gel, and an ionic gel. Preferably each of the substance sections comprises the same substance.

Preferably, at least two of the substance sections are positioned adjacent to an electrode Preferably, at least one substance section is positioned adjacent to an anode and at least one substance section is positioned adjacent to a cathode.

Preferably, the capsule comprises two substance sections, wherein a first substance section is positioned adjacent to an anode and a second substance section is positioned adjacent to a cathode.

Preferably, the capsule comprises a securing and/or placement structure for positioning the capsule in the ion diffuser. Preferably, the capsule comprises a magnet.

According to another aspect of the present invention, there is described a method of operating an ion diffuser as defined in claim 13.

Preferably, the method comprises providing the current so as to cause the emission of ions in a plasma state.

Preferably, the method comprises detecting and/or identifying a substance and/or a capsule in the cavity.

Preferably, the method comprises operating the ion diffuser in dependence on one or more of: a user input; a sensor reading; and a substance and/or capsule located in the cavity, preferably a type of the substance and/or capsule and/or a capacity of the substance and/or capsule.

According to another aspect of the present invention, there is described a method of manufacturing a capsule for use in an ion diffuser as defined in claim 14.

Preferably, the method comprises placing a substance in the capsule.

Preferably, the substance comprises an ionic gel.

Preferably, forming the substance comprises mixing a matrix (e.g. a polymer matrix) with an ionic liquid and/or inserting an ionic liquid into a matrix.

### Brief description of the Drawings

The disclosure will now be described, purely by way of example, with reference to the accompanying drawings.
Figures 1a, 1b, 1c, and 1d shows an ion diffuser according to the present invention.
Figures 2a, 2b, 2c, and 2d show a capsule that may be used with the ion diffuser.
Figure 3 shows an example of a capsule not falling under the present invention.
Figure 4 shows a flowchart for a method of generating ions using the ion diffuser.
Figure 5 shows a flowchart for a more detailed method of generating ions using the ion diffuser.
Figure 6 shows a circuit that may be included in the ion diffuser.

### Description of the preferred embodiments

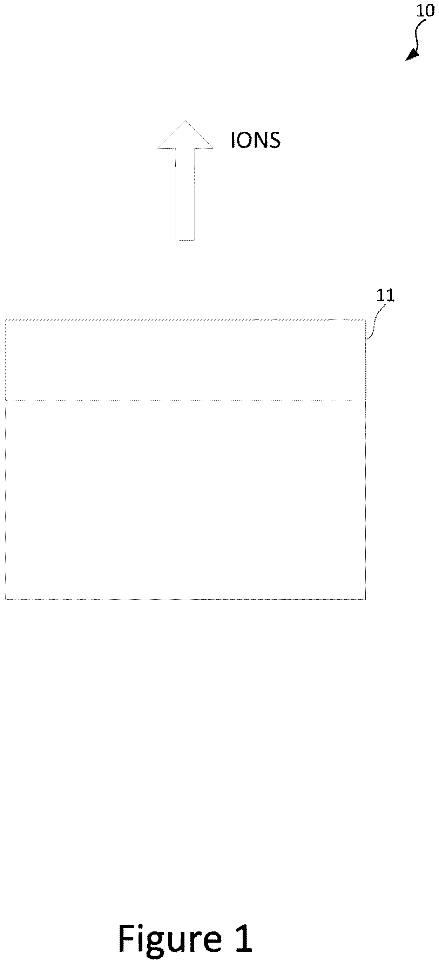

Referring to Figure 1a, there is described an ion diffuser 10. The ion diffuser is arranged to provide, e.g. produce, ions. These ions may interact with particulate matter in the air such that the particulate matter becomes charged. This charged particulate matter can then be attracted to a charged plate (or simply to existing walls and ceilings of a structure) and thereby removed from the air. Equally, the ions may interact with matter in the air to damage and/or inactivate this matter (e.g. to destroy viruses). Therefore, the ion diffuser may be used as an air purifying device.

The ion diffuser 10 of the present invention comprises a cavity 11 that is arranged to receive a substance. Typically, the cavity is arranged to receive a capsule, which capsule comprises the substance. The substance comprises ions and/or that enables the production of ions. The use of such a substance enables ions to be produced using a comparatively low energy input (as compared to conventional air ionisers). Therefore, the ion diffuser can be provided as a portable device.

The cavity 11 may comprise a canister into which the capsule can be placed. Typically, the capsule sits inside the cavity and is supported by a floor of the ion diffuser 10. Typically, the ion diffuser comprises a securing structure arranged to secure the capsule. For example, the ion diffuser may comprise a clip. Equally, a capsule may be secured in the ion diffuser using a bayonet mechanism, a magnet (e.g. the ion diffuser and the capsule may each comprise a magnet and/or an electromagnet), an interference fit, or a latch.

Equally, the substance may be placed directly into the cavity 1. Since the substance is an ionic gel, the substance may be poured into the cavity The present invention relates to an ion diffuser 10 that is provided with a substance. The substance comprises ions; this enables the ion diffuser to provide ions with a small power input and also enables a user to alter the ions provided by changing the substance in the cavity 11.

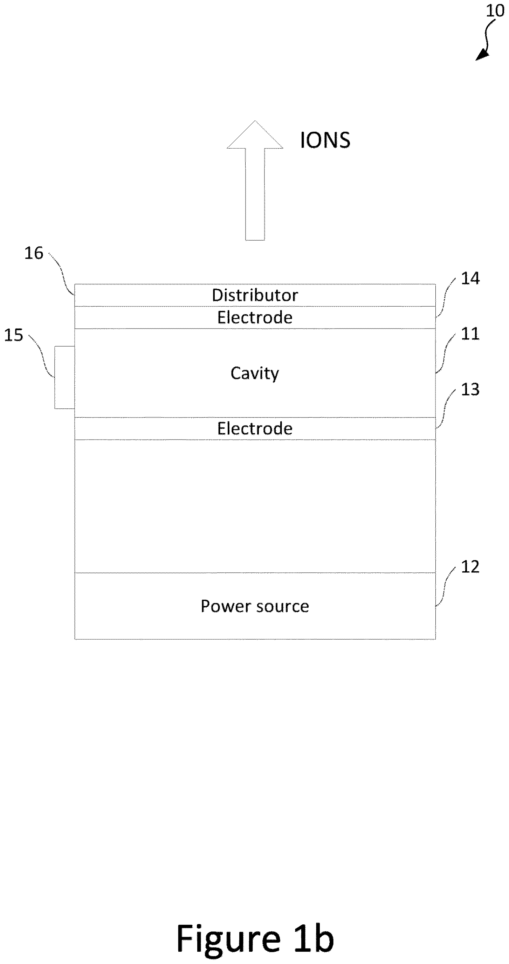

Referring to Figure 1b, there is described a more detailed embodiment of the ion diffuser 10 that comprises a number of optional features. It will be appreciated that these features may be provided in any combination without departing from the scope of the claims.

Specifically, the ion diffuser 10 of Figure 1b comprises: a power source 12; one or more electrodes 13, 14; a placement structure 15; and a distribution mechanism 16.

The power source 12 is arranged to provide power to the electrodes 13, 14, and/or another component of the ion diffuser 10. As an example, the ion diffuser may comprise a user interface, a sensor, or a processor and/or a microcontroller that is powered using the power source. Such components enable the ion diffuser to interact with users and/or the environment; for example, the ion diffuser may be arranged to produce ions in dependence on a user input and/or an environmental condition, such as the detection of particulate matter in the air.

The power source 12 typically comprises a battery, so that the ion diffuser can be provided as a portable device. The battery may be a rechargeable battery, which may be recharged using a universal serial bus (USB) connection. Equally, the power source may comprise a mains connection so that the ion diffuser can be plugged into a plug socket. The power source may receive a voltage of less than 20V, less than 10V, and/or 5V DC. The power source may be arranged to provide one or more of: a power of at least 4 Watts; a power of no more than 15 Watts; and a power of 10 Watts.

The power source 12 may comprise a transformer that is arranged to provide a high voltage. This allows the ion diffuser 10 to provide a high-voltage spark to a capsule located in the cavity 11. The transformer is typically arranged to provide a voltage of at least 10000 Volts, at least 15000 Volts, and/or at least 20000 Volts

The electrodes 13, 14 comprise an anode and/or a cathode. Typically, the electrodes comprise both of an anode and a cathode. The anode is arranged to release electrons to the power source 12 and the cathode is arranged to receive electrons from the power source. Therefore, positively charged ions (anions) are attracted to the anode and negatively charged ions (cations) are attracted to the cathode. In this way, the anode and the cathode may be used to break down a substance and/or to collect ions near the electrodes. These ions can then be provided to the environment surrounding the ion diffuser 10.

The electrodes 13, 14 and/or, a subset of the electrodes, may be located in the capsule (in the cavity 11), so that the ion diffuser 10 may be provided without one or both of these components.

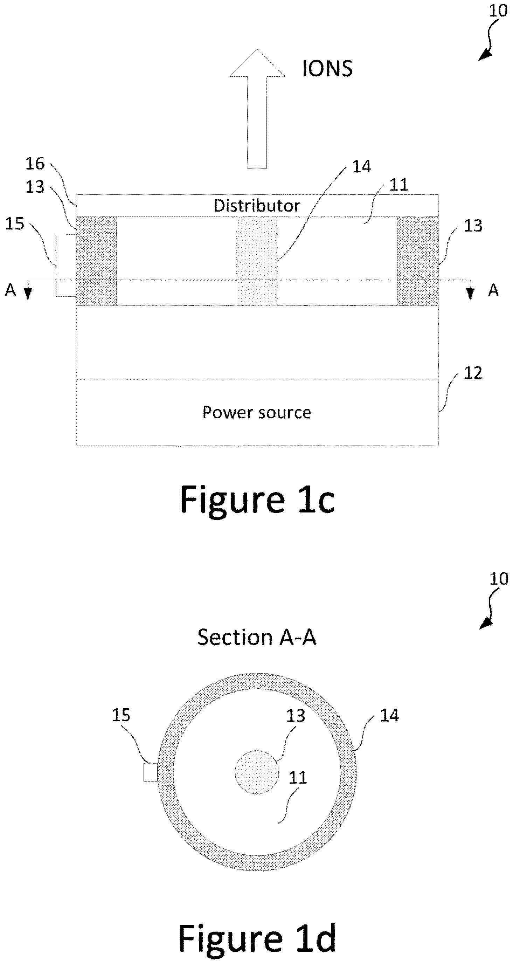

It will be appreciated that the electrodes 13,14 may be positioned in a variety of arrangements. For example, the electrodes may be placed to either side of the cavity as shown in Figure 1b. Equally, one of the electrodes may be located in the interior (e.g. the centre) of the cavity and the other electrode may be located about the exterior of the cavity as shown in Figures 1c and 1d.

The electrodes 13, 14 may be formed of carbon or a similar inert substance so that they do not react with ions provided by the ion diffuser 10. In some embodiments, the electrodes are formed of copper.

The placement structure 15 is arranged to ensure that a capsule is correctly positioned in the cavity. The placement structure 15 may comprise an indicator, that is useable to check the positioning of the capsule and/or a structure that encourages the correct placement of the capsule. Typically, the placement structure comprises a notch that is arranged to receive a part of the capsule. In some embodiments, the placement structure comprises a magnet that is arranged to attract (or repel) a corresponding magnet in a capsule.

The distribution mechanism 16 is arranged to distribute the ions produced by the ion diffuser 10. The distribution mechanism may comprise a fan. Equally, the distribution mechanism may comprise a charged plate that is arranged to repel the ions.

It will be appreciated that the use of an active distribution mechanism is optional; ions may instead be distributed by diffusion and/or Brownian motion.

The ion diffuser 10 may also comprise a capsule identifier (and/or a substance identifier) that is arranged to identify a capsule and/or determine a characteristic of a capsule. In particular, the capsule identifier may determine one or more of:
- A capacity of a capsule and/or the volume of the substance remaining in the capsule and/or the cavity 11. The capsule identifier may then output an indication when the capsule needs replacing (e.g. when it is nearly out of ions).
- A type of a substance and/or capsule. The capsule identifier may display a type of capsule, where a capsule may be arranged to be particularly effective for a certain purpose (e.g. removing viruses from the air or removing bacteria from the air). Equally, the capsule identifier may be arranged to identify authorised capsules, where the ion diffuser 10 is arranged to operate only when an authorised capsule is inserted. This may comprise the anode 13 and the cathode 14 only being operable when an authorised capsule is inserted. This prevents a capsule being used that could damage the ion diffuser.

In some embodiments, the ion diffuser 10 further comprises a charged plate that is arranged to attract charged matter. This enables the ions to interact with particulate matter in the air so as to impart a charge to that particulate matter. The charged particulate matter can then be attracted to the charged plate on the ion diffuser.

Typically, the electrodes 13, 14 comprise both an anode and the cathode, which are used to perform bipolar ionisation. Specifically, the ion diffuser 10 provides both positive ions (anions) and negative ions (cations). In contrast, in some embodiments, the ion diffuser may be arranged to provide only anions and/or only cations. With this arrangement, the electrodes are typically arranged so that a current flows between the electrodes via the capsule.

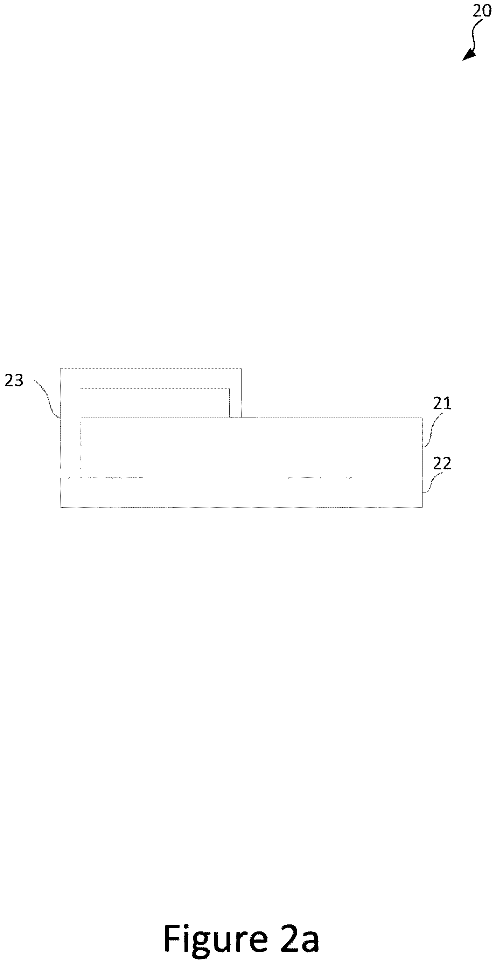

The ions produced by bipolar ionisation may help to neutralise viruses by interacting with hydrogen in the protein coat of a virus and thereby damaging the structure of the virus. Referring to Figure 2a, there is described a capsule 20 that may be used with the ion diffuser 10 of Figure 1a or 1b.

The capsule 20 comprises a substance 21. In this embodiment, the capsule also comprises one or more electrodes 22, 23. Such a capsule may be used with an ion diffuser 10 that does not comprise electrodes, where the electrodes of the capsule may still be powered using the power source 12 of the ion diffuser. Equally, the capsule may comprise a power source, such a power source may be arranged to lose power as the substance runs out.

In this embodiment, a first electrode 23 is provided as a needle. This provides a point from which a spark may be produced and transmitted to the substance 21 in order to release ions from the substance.

The substance 21 is typically a substance that contains ions and/or from which ions can easily be produced. According to the invention, the substance 21 comprises an ionic gel. An ionic gel comprises an ionic liquid that is held by an inorganic or polymer matrix. This provides a material with a high ionic conductivity in a solid state.

An ionic gel typically comprises an ionic solution trapped in a crystal lattice of a polymer to provide a mixture of an ionic liquid and a polymer. When the capsule 20 is filled with such a gel, the polymer reacts with air and forms a porous material within which the ionic solution is absorbed. The polymer itself does not interfere with the operation of the liquid ionic solution. The substance 21 typically comprises a material (e.g. an element) with an atomic number greater than 1 (e.g. greater than that of Hydrogen), and/or greater than 8 (e.g. greater than that of Oxygen). In some embodiments, an element present in the substance has an atomic number greater than 10, greater than 25, and/or greater than 50.

Typically, the substance 21 comprises a metal. The substance may comprise one or more of: Hydrogen, Sodium, Magnesium, Silver, Chlorine, Platinum, Gold, Zinc, Molybdenum, Iodine, and Potassium. Typically, the substance comprises a non-toxic material.

The substance 21 may comprise one or more of:
- A metal (e.g. an alkali metal, an alkaline earth metal, and/or a transition metal).
- A halogen.
- A compound of a halogen and/or a metal
- A compound comprising a metal.
- One or more of: Magnesium, Silver, Platinum, Gold, and potassium.
- A liquid acrylic polymer.

Where the substance comprises a compound, typically one of the component elements of the compound has an atomic number greater than 8 (e.g. Na₁₁, Mg₁₂, or Ag₄₇). In some embodiments, an element present in the substance has an atomic number greater than 10, greater than 25, and/or greater than 50).

Where the substance comprises a compound, this involves the substance 21 comprising an ionic compound. For example, Silver Nitrate is formed of a compound of silver ions (Ag+) and Nitrate ions (NOs-). Silver Nitrate is soluble, so that the liquid ionic solution may comprise a solution of Silver Nitrate, which solution comprises separate Ag+ and NO₃-ions. The addition of energy to this compound (e.g. via a spark) is useable to convert the Silver Nitrate to a plasma. In this form, the Silver ions and the Nitrate ions remain separated and are able to separately interact with, and break down, particles in the air (such as viruses). Furthermore, in contrast to in the liquid state, in the plasma state the ions are able to diffuse into the surroundings of the ion engine 10. Thus the input of energy to this substance provides ions. It will be appreciated that Silver Nitrate is used as an example here, and that similar processes occur with other substances.

The ions produced may be ions of a single element (e.g. silver ions Ag+) and/or ions of a compound (e.g. a molecular compound), such as nitrate ions (NOs-). More generally, the ion engine 10 is arranged to emit charged particles, where the ion engine is typically arranged to emit both positively charged particles and negatively charged particles.

Typically, at least a part of the substance 21 is liquid at room temperature. In some embodiments, at least a part of the substance has a melting point of less than 135 degrees Celsius, less than 100 degrees Celsius, less than 50 degrees Celsius, and/or less than 20 degrees Celsius.

Typically, the substance 21 is stable; in particular, the substance may be formed so that it does not react with water or air and so that it does not evaporate under normal conditions.

Substances with a higher atomic number than Oxygen provide ions with a weight greater than the weight of Oxygen ions. This increased weight can lead to increased penetration, and an increased ability of these ions to break down harmful matter, such as viruses. Furthermore, the use of the substance 21 may enable the provision of ions with a longer lifetime than those Oxygen and Nitrogen ions provided by conventional air purifiers.

In some embodiments, the substance 21 is arranged to provide free radicals and/or hydroxl free radicals (˙OH) and/or hydroxl ions (OH-).

The manufacture of the substance 21 typically comprises a matrix being mixed with an ionic liquid. The manufacture of the capsule 20 typically comprises the substance 21 being placed into the capsule.

More specifically, the manufacture of the substance 21 typically comprises the preparation of a liquid ionic solution, which is then encapsulated in a gel and/or solid. For example, the liquid ionic solution may be exposed to air so that it forms a gel (where the liquid ionic solution is trapped in the pores of a solid material - i.e. the liquid ionic solution is held in a matrix). In order to release the ions of the liquid ionic solution from the gel, a barrier potential must be overcome, e.g. a threshold amount of energy must be provided. This energy is provided using the electrodes. The application of energy to the substance causes a change in the state of the ionic solution to a plasma. This plasma, which comprises the ions, is able to escape from the pores of the ionic substance and thereby diffuse into the environment surrounding the ion engine.

The energy needed to overcome the barrier potential and to convert the liquid ionic solution to a plasma is less than the energy needed to create the ions in the first place, so that, for example, ions of silver may be created using an electrical signal of high power and then encapsulated in the substance 21 - and these ions can later then be released by applying only a low power (e.g. of 10W). Equally, the initial production of the liquid ionic solution may require complex chemical reactions, where the ions can then be emitted from the substance based on the application of a low power. This enables the ion engine to be provided as a portable device with only a small power supply while still being capable of releasing a range of ions, including heavy ions.

In this regard, the ions stored in the substance 21 may be ions of elements and/or ions of compounds. Typically, where ions of compounds are provided, the compounds comprise a reasonably heavy element (e.g. an element with an atomic number greater than 8).

Typically, the ion engine 10 and the substance 21 are arranged so that the ion engine emits a plasma. In particular, a high voltage is applied to the capsule 20 in order to transform a portion of the substance into a plasma and/or to emit plasma ions from the ion engine.

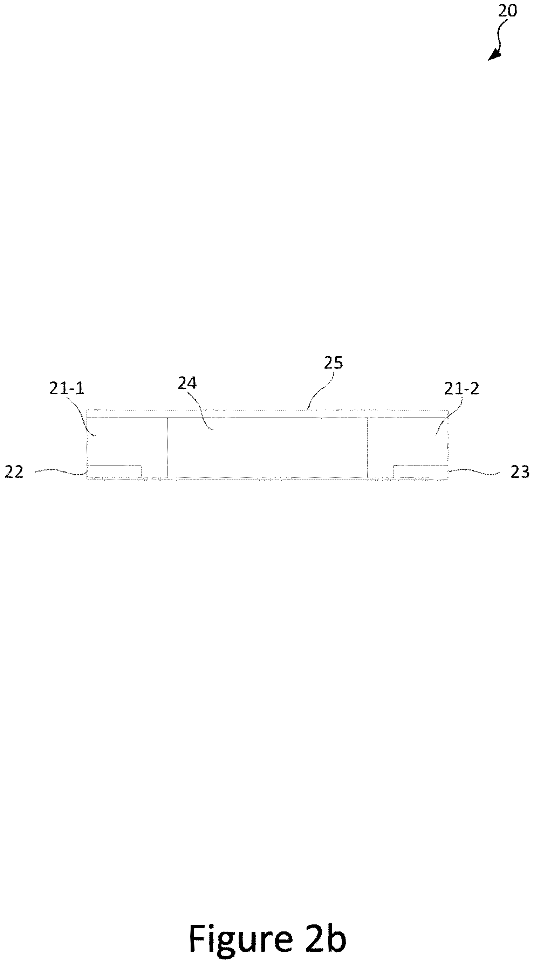

Referring to Figure 2b, there is shown an embodiment of the capsule 20 in which the substance is provided in a plurality of substance sections 21-1, 21-2 separated by an air gap 24. Each of the substance sections is located adjacent an electrode 22, 23. These electrodes may each be anodes (where a cathode is provided in the ion diffuser 10), each be cathodes (where an anode is provided in the ion diffuser), and/or the electrodes may comprise at least one anode and at least one cathode.

Typically, the ion diffuser 10 is arranged to provide bipolar ionisation, where both positive and negative ions are produced. This may comprise one of the substance sections 21-1, being located adjacent an anode 22, and the other substance section 21-2 being located adjacent a cathode 23.

The electrodes 22, 23 provide electricity (e.g. a spark) to the substance sections 21-1, 21-2. This generates ions, which are typically emitted into the air gap 24 and then distributed by the ion diffuser 10.

The capsule 20 comprises a lid 25; typically, the lid comprises perforations or holes to aid the emission of ions. The lid may also comprise, or interact with, the distribution mechanism 16. The use of the lid may enable the directional emission of ions, where the other walls of the capsule may be arranged to block the emission of ions.

The air gap 24 is typically greater than 10mm, greater than 20mm, greater than 30mm, and/or greater than 30mm. The air gap 24 is typically less than 50mm, greater than 40mm, and/or less than 30mm. Typically, the air gap is in the range of 20mm - 30mm.

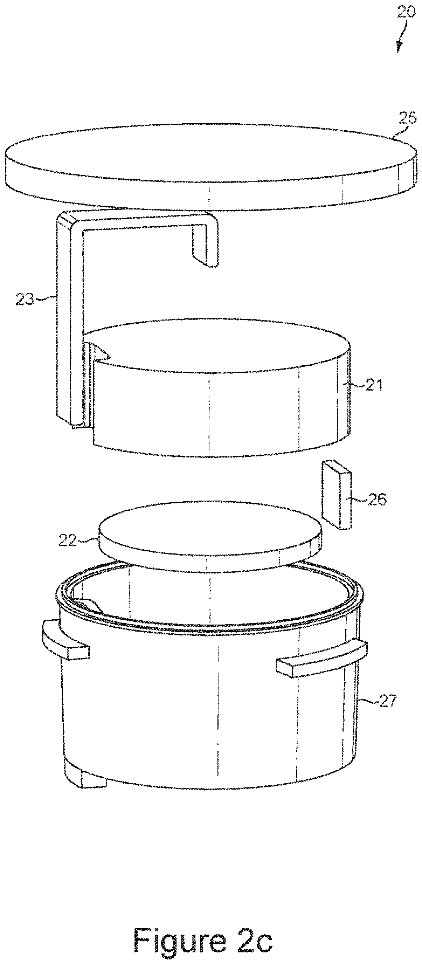

Referring to Figure 2c, an exploded view of an embodiment of the capsule 20 is shown. The substance 21 in this embodiment is shown as a single block of material, it will be appreciated that this substance could be split into a plurality of substance sections as described with reference to Figure 2b.

This embodiment further comprises a positioning structure 26, which is arranged to ensure correct positioning of the capsule 20 in the ion diffuser 10. This positioning structure may, for example, comprise a magnet or an extended portion of the capsule that is arranged to fit into a notch of the ion diffuser. This positioning structure may also comprise an identifier, which provides the ion diffuser with information about a characteristic of the capsule (e.g. the type of substance within the capsule (equally, the capsule may comprise a separate identifier).

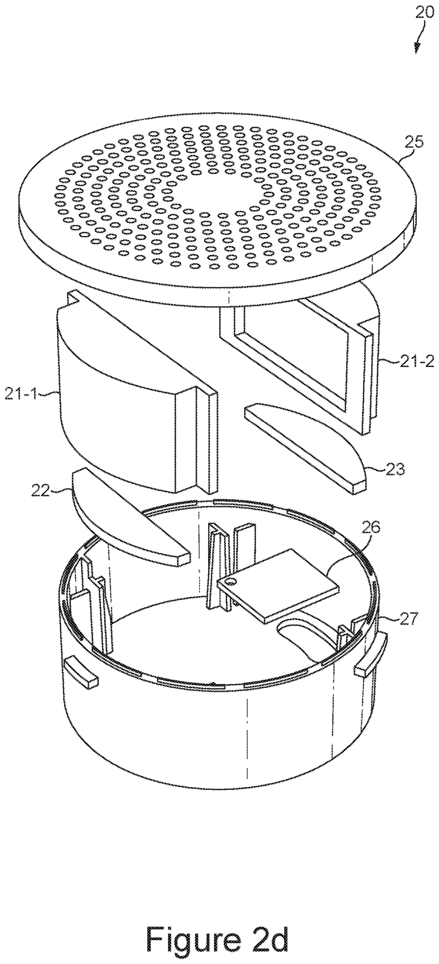

In this embodiment, the capsule 20 comprises a casing 27. The casing holds and protects the other components of the capsule and typically comprises a hard and/or tough material, such as a tough plastic. The casing is arranged to fit into the ion diffuser 10 and may be arranged to interact with the power source 12 of the ion diffuser so as to provide power to the electrodes. Referring to Figure 2d, an exploded view of another embodiment of the capsule 20 is shown. In this embodiment, the capsule 20 comprises a plurality of substance sections 21-1, 21-2 in which the substance is located. Each substance section is located adjacent one of the electrodes 22, 23.

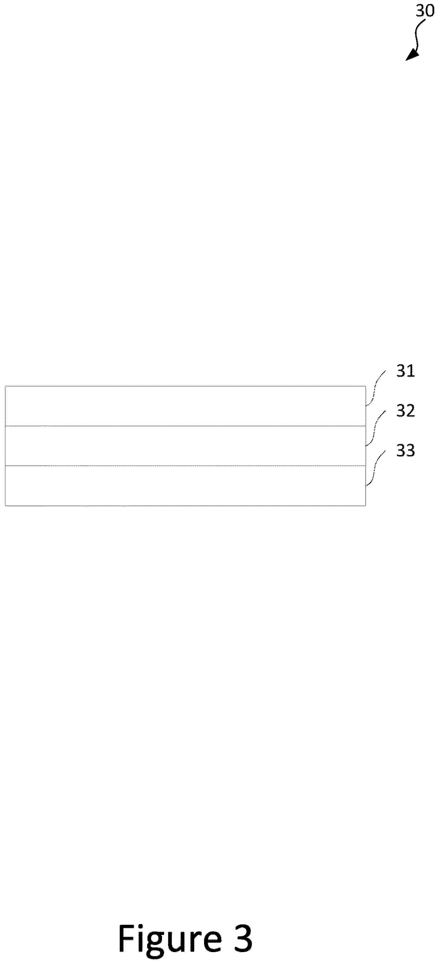

An exemplary composition of a substance 21 not covered by the present invention is now described with reference to Figure 3.

Referring to Figure 3, in a first example 30 of the capsule 20, the substance 21 is formed of three layers. Typically, the first layer 31 comprises a sea salt; the second layer 32 comprises a salt; and the third layer 33 comprises a potassium salt.

In a specific example not falling under the invention, the capsule 20 comprises a tablet of salts comprising of (or consisting of) three parts:
1. a sea salt with a layer of herbal extracts;
2. a salt layer with clay, optionally 1 mm thick; and
3. a potassium salt layer with 3 percent Iodine solution.

The diameter of the capsule 20 may be 25mm and/or the total thickness may be 10 mm. Such a capsule may be sufficient for use in a living space of up to seventy square meters for a duration of one month. In some embodiments, the capsule may be arranged to be used daily for up to two hours with an activation period of three minute that occurs at twenty minute intervals using a timer control function. The capsule may be replaceable and/or may be useable for at least thirty days.

In various embodiments, the substance 21 comprises one or more of: platinum, silver, gold, magnesium, and potassium. The use of different materials enables the provision of different ions (e.g. platinum, silver, gold, magnesium, and potassium ions may be emitted).

Beneficially, since the ion diffuser 10 is not arranged to ionise air, no substantial amount of ozone is produced as a by-product of the ionisation. This enables the ion diffuser to be used in areas where conventional air ionisers are not useable (e.g. in enclosed spaces where living things are present, such as in vehicles).

The capsule 20 may comprise a power source, e.g. a rechargeable power source. This power source may be used to provide electricity (e.g. a voltage and/or current) to one of the electrodes 13, 14 and/or to provide information about the capsule.

The capsule 20 typically comprises an identifier arranged to provide a characteristic of the capsule. The identifier may provide the characteristic visually (e.g. as a coloured sticker) and/or may provide the characteristic via a communication interface. As examples, the capsule may comprise a radio frequency identifier (RFID) interface and/or a near field communication (NFC) interface.

The characteristic may, for example, comprise a remaining volume of the substance 21 within the capsule 20 and/or a type of the substance within the capsule.

In some embodiments, the ion diffuser 10 includes the capsule 20, where the capsule may be permanently fixed within the cavity 11.

Typically, the cavity 11 is arranged so that a number of different, interchangeable, capsules may be used. The substance located within each of the interchangeable capsules may differ depending on a purpose of that capsule. For example, a first capsule/substance may be advantageous for removing bacteria from the air and a second capsule/substance may be advantageous for removing viruses from the air.

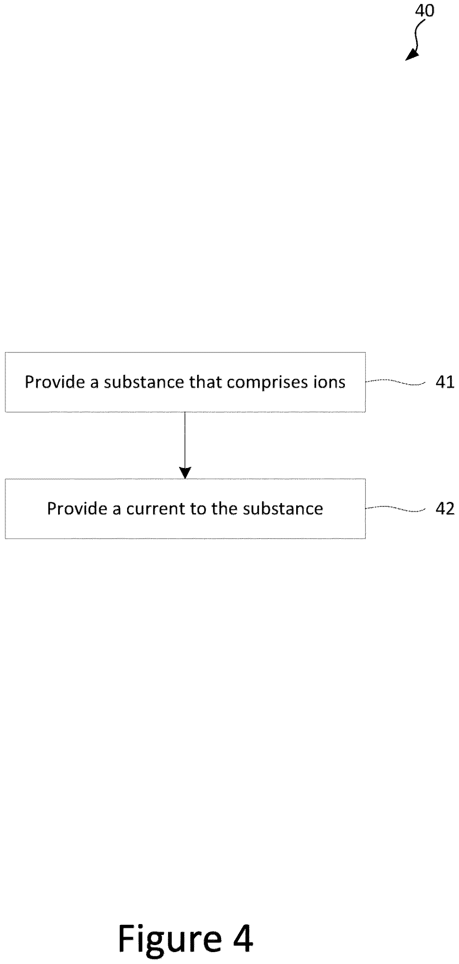

In some embodiments, the substance 21 is arranged to provide the electrodes. In particular, there may be provided an ionic gel that is useable as one or more of: a cathode, and an anode. Referring to Figure 4, there is described a method 40 of generating ions using the ion diffuser 10. Any combination of one or more of these steps may be performed by the ion diffuser.

In a first step 41, a substance is provided that comprises one or more ions (e.g. this substance may comprise an ionic gel). This first step typically comprises the substance being placed into the cavity 11 of the ion diffuser 10.

In a second step 42, electricity (e.g. a current and a voltage) is provided to the capsule 20 (and to the substance 21) so that ions are released from the substance. Typically, this comprises the ion diffuser 10 providing a voltage/current that is sufficient for electrons in the substance to overcome a potential barrier relating to an ionisation potential. This results in the generation of ions, which ions are then distributed into a surrounding environment (e.g. via diffusion or via the distribution mechanism 16.

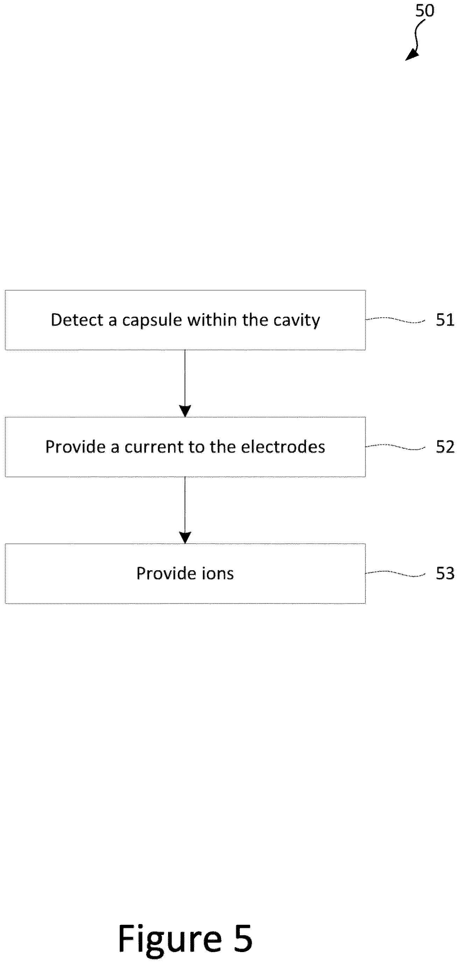

Referring to Figure 5, there is described a more detailed method 50 of generating ions using the ion diffuser 10. Any combination of one or more of these steps may be performed by the ion diffuser.

In a first step 51, the ion diffuser 10 detects the capsule 20 in the cavity 11. This may comprise detecting the correct positioning of the capsule using the positioning structure 15 and/or detecting a characteristic of the capsule, such as a remaining volume of the substance 21 in the capsule.

The characteristic may be used to determine a voltage/current to provide to the electrodes (where the voltage/current that is suitable may depend on the substance 21 in the capsule).

In some embodiments, the characteristic is displayed to a user (e.g. using a display screen of the ion diffuser 10). This enables the user to identify the capsule 20 that is positioned in the ion diffuser and, for example, to see a remaining volume of the substance 21 in the capsule. The ion diffuser may then indicate to the user when the capsule should be replaced and/or changed.

In a second step 52, the ion diffuser 10 provides electricity (e.g. a current and a voltage) to the electrodes 13, 14 using the power source 2. This typically comprises providing a high voltage that causes electricity (e.g. a spark) to pass through the substance 21. For example, the power source may provide a voltage of 14kV. The power source may provide only a small current, e.g. the power source may provide a voltage of 14kV at a power of 10W. Equally, a continuous current may be passed through the substance. The use of a (brief) spark enables a high voltage to be provided with a portable power source (that has a low Wattage).

In a third step 53, ions are provided by the ion diffuser 10. When the spark passes through the substance 21 in the capsule 20, anions in the substance are attracted to the anode 13 and/or cations in the substance are attracted to the cathode 14. This leads to ions being emitted from the substance; these ions can then be emitted by the ion diffuser, e.g. using the distribution mechanism 16.

In examples, not covered by the invention, the substance 21 comprises a salt. The salt may be heated in order to provide an ionic liquid and in order to provide ions, where this heating may occur using the electrodes 13, 14 and/or using a separate heating source such as a heater.

In some embodiments, the electrodes 13, 14 and the substance 21 are arranged so that a plasma is formed in the capsule 20 wherein the voltage/current is provided to the electrodes. Ions are emitted by this plasma and provided by the ion diffuser 10 to a surrounding environment.

An example of the use of the ion diffuser 10, not covered by the present invention, is now described considering the use of a capsule comprising a potassium salt Potassium Iodide (KI). The potassium Iodide may be heated until it is molten before a current is provided to the electrodes 13, 14 (or may be heated using resistive heating by providing a current to the electrodes).

When electricity is provided to the electrodes, ions of potassium (K+ cations) are attracted to, and gather at, the cathode 14 and ions of Iodine (I- anions) are attracted to, and gather at, the anode 13. These ions can then be emitted by the ion diffuser 10; the emitted ions are then able to interact with particulate matter in the air in order to purify the air.

It will be appreciated that varying the composition of the substance 21 enables the production of different ions.

In some embodiments, the ion diffuser 10 comprises a filter, where a subset of the produced ions are blocked from emission by the filter. In practice, small amounts of Iodine may have a beneficial effect on health but it can be undesirable to release large amounts of Iodine. Therefore, an amount of the Iodine may be blocked from emission (e.g. using the filter). Typically, the ions produced are in a gaseous form so that these ions diffuse to fill the environment in which the ion diffuser 10 is located. In some embodiments, the ions produced may be in a liquid state, where the distribution mechanism 16 may be arranged to distribute these ions as an aerosol. In order to do this, the distribution mechanism may comprise a source of pressurised gas.

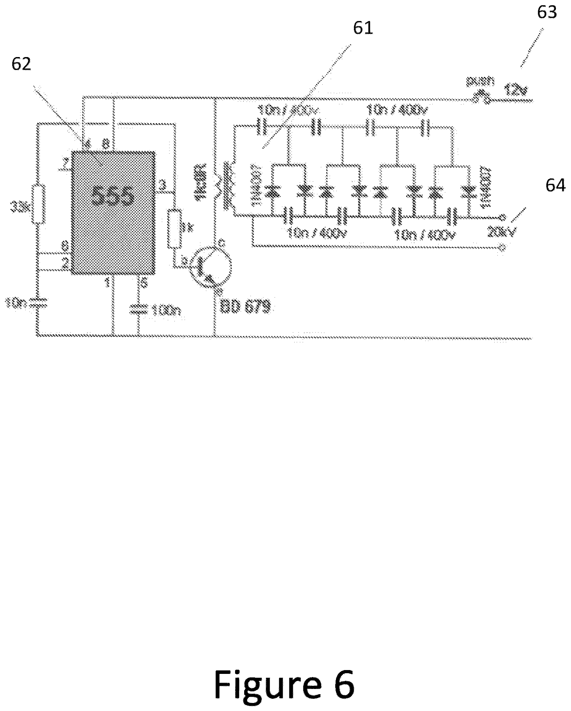

Referring to Figure 6, there is shown an exemplary circuit that may form a part of the ion diffuser. Specifically, the exemplary circuit comprises a transformer 61, a microcontroller 62, a switch 63, and a circuit output 64.

The transformer 61 receives an input voltage from the power source 2 and steps up the input voltage to provide an output voltage to the circuit output 64, which provides the output voltage to the anode 13.

The microcontroller controls the transformer 61 and/or other components of the ion diffuser 10. The switch 63 enables the ion diffuser 10 to be turned on or off.

Typically the behaviour of the ion diffuser 10 (e.g. of the microcontroller 62) depends on one or more of:
- A user input. The user may be able to turn the ion diffuser 10 on or off and/or to control a frequency of ion emission, a duration of ion emission and/or an intensity of ion emission. These parameters may be controlled by controlling the output voltage that is provided to the electrodes 13, 14.
- A sensor reading. The ion diffuser 10 may comprise a sensor, which may detect a feature of an environment in which the ion diffuser is located. For example, the sensor may detect the entry or exit of a person from a room, a cleanliness of an environment, and/or the concentration of a particle in the air.
- The capsule 20 located in the cavity 11. Different capsules may have different operating conditions; for example, the ideal frequency of emission of ions and/or the voltage required to emit ions may depend on the substance 21 in the capsule.

In some embodiments, the ion diffuser is arranged to be controlled remotely. Therefore, the ion diffuser 10 may comprise a communications interface, which may be wired or wireless. As examples, the ion diffuser may comprise a Bluetooth interface, an area network (e.g. ethernet) interface, and/or a 3G, 4G, and/or 5G interface. In some embodiments, the communications interface comprises a Zigbee and/or Zwave interface.

In some embodiments, the ion diffuser 10 is arranged to be monitored and/or controlled by a computer application. This enables a user to control the operation of the ion diffuser remotely (e.g. to activate the ion diffuser before returning home). Furthermore, this enables the ion diffuser to be controlled by other apparatuses on a network, which other apparatuses may comprise relevant sensors - for example, a smartphone may be arranged to communicate with the apparatus based on location, so that the ion diffuser is active when the smartphone is in the vicinity of the ion diffuser.

### Test results

The ion diffuser 10 is typically arranged to purify the air from bacteria and viruses. Testing of an embodiment of the ion diffuser has shown suppression of total microbiological count (CFU) in Petri dishes installed in different positions within a testing space. On average, air purification of 63 - 90 % was achieved, depending on the composition of the substance 21. Below are described the test results with various consistencies of emissions.

The tests were performed in a room with an area of 15 square meters and a volume of 45 cubic meters, where Petri dish samples were located inside the room. A first sample was used as a control sample. A second sample was located on a table near the ion diffuser 10. A third sample was located on a chair at a distance from the ion diffuser.

A first test was performed in a clean room; the test results for this first test are given in Table 1 below:

**Table 1**

| Parameter | Unit of measurement | Result | Uncertainty | Test method |
|---|---|---|---|---|
| **Sample 1** - **control sample** | | | | |
| Total number of microorganisms | CFU/1.5h | 52 | - | T-261-21:2010 |

| **Sample 2** - **a sample on the table** | | | | |
|---|---|---|---|---|
| Total number of microorganisms | CFU/1.5h | 19 | - | T-261-21:2010 |

| **Sample 3 - a sample on the chair** | | | | |
|---|---|---|---|---|
| Total number of microorganisms | CFU/1.5h | 21 | - | T-261-21:2010 |

A second test was performed in a polluted room; the room was extensively used by workers during the test to cause pollution. The test results for this second test are given in Table 2 below.

**Table 2**

| Parameter | Unit of measurement | Result | Uncertainty | Test method |
|---|---|---|---|---|
| **Sample 1 - control sample** | | | | |
| Total number of microorganisms | CFU/2h | 245 | - | T-261-21:2010 |

| **Sample 2 - a sample on the table** | | | | |
|---|---|---|---|---|
| Total number of microorganisms | CFU/2h | 80 | - | T-261-21:2010 |

| **Sample 3 - a sample on the chair** | | | | |
|---|---|---|---|---|
| Total number of microorganisms | CFU/2h | 91 | - | T-261-21:2010 |

In order to purify the air, the ion diffuser 10 typically generates active ions during use. As shown by the test results above, 67% - 90% of purification was achieved within two hours of exposure using the ion diffuser.

The purification achieved depended on the initial contamination levels, as well as on the ongoing intentional contamination occurring during the exposure, modelling a real-life situation. Provided there is a fixed level of initial contamination, the ion diffuser 10 has been shown to reduce the pollution in surrounding air by up to 99%.

The ion diffuser 10 may provide the following benefits:
1. The ion diffuser 10 acts to eliminate viruses and bacteria making it a cheap and convenient solution in fighting Coronavirus (COVID 19) and other viruses.
2. The ion diffuser 10 is non-toxic and not poisonous.
3. The ion diffuser 10 does not require air to be pumped through the apparatus like many other existing ionising devices; this pumping can collect dust and reducing the work area (as in UV lamp based devices).
4. Ions fill up the air in a given space evenly, working on the total volume at once.
5. The ion diffuser 10 purifies air without people needing to vacate the premises being purified.
6. The ion diffuser 10 consumes only a small amount of energy enabling it to be provided as a portable device.
7. The ion diffuser 10 is scalable to smaller and bigger dimensions depending on the environment in which the ion diffuser is used.
8. The ion diffuser 10 is easy and cheap to manufacture.
9. The ion diffuser 10 device is safe to operate.

### Alternatives and modifications

It will be understood that the present invention has been described above purely by way of example, and modifications of detail can be made within the scope of the invention as defined by the appended claims.

In some embodiments, the cavity 11 may be arranged to receive the substance 21 directly (e.g. a user may be able to pour or place the substance into the cavity). The ion diffuser may then provide a current to this substance using electrodes positioned about the cavity. It will be appreciated that operations described in the detailed description as being performed on the capsule (e.g. the provision of a current) may equally be performed directly on the substance. In some embodiments, the ion diffuser 10 is arranged to receive a plurality of substances and/or capsules, where the ion diffuser is able to provide a current to a subset of the substances or capsules. For example, a user may insert capsules for a plurality of purposes (e.g. anti-viral and anti-bacterial) and then select a capsule to which current should be provided. This selection may be altered later; for example, the ion diffuser may be arranged to periodically alter the substance(s) to which the current is provided.

Typically, the ion diffuser 10 comprises a plurality of cavities, for example the cavity 11 shown in Figures 1a- 1d may be divided into a plurality of smaller cavities using internal walls, where these smaller cavities are each able to receive a substance and/or a capsule. The ion diffuser may also comprise a plurality of electrodes, where these electrodes can be used to selectively provide a current to a substance/capsule in any of the smaller cavities. This arrangement enables the user to switch out the capsule less frequently and/or to use the ion diffuser for a plurality of purposes without inserting a new capsule.

Reference numerals appearing in the claims are by way of illustration only and shall have no limiting effect on the scope of the claims.

## Claims

1. An ion diffuser (10) for generating ions, the ion diffuser (10) comprising:
a cavity (11) arranged to receive a substance;
the substance being located in the cavity; and
a power source (12) arranged to provide a current to the substance to cause the emission of ions from the substance;
wherein the substance comprises an ionic gel, the ionic gel comprising an ionic liquid that is held by an inorganic or polymer matrix.

2. The ion diffuser (10) of claim 1, wherein the ion diffuser (10) is arranged to generate positive ions and/or both positive and negative ions.

3. The ion diffuser (10) of any preceding claim, wherein the substance comprises a material and/or element with an atomic number of greater than 8.

4. The ion diffuser (10) of any preceding claim, wherein:
the ion diffuser (10) is arranged to receive a plurality of different types of substances; and/or
the ion diffuser (10) comprises a plurality of cavities arranged to receive a plurality of substances, wherein the ion diffuser (10) is arranged to selectively provide a current to one or more of the substances, preferably based on a user input; and/or
the ion diffuser (10) is arranged to identify a type of the or each substance.

5. The ion diffuser (10) of any preceding claim, being arranged to receive a capsule (20) comprising the substance (21), preferably wherein:
the cavity (11) is arranged to receive the capsule (20); and/or
the ion diffuser (10) is arranged to receive a plurality of capsules, preferably wherein the plurality of capsules comprise different substances;
more preferably, wherein:
the cavity (11) is adapted to receive a capsule (20) comprising one or more electrodes (22, 23), yet more preferably wherein the cavity (11) is arranged to receive a capsule (20) comprising an anode (22) and/or a cathode (23); and/or
the ion diffuser (10) is arranged to identify a type of the or each capsule (20).

6. The ion diffuser (10) of any preceding claim, wherein:
the ion diffuser (10) comprises one or more electrodes (13, 14) connected to the power source (12), preferably an anode and/or a cathode; and/or
the power source (12) is arranged to provide a current to an electrode (22, 23) of a/the capsule (20) and/or wherein the power source (12) is arranged to provide a spark to the substance; and/or
the power source (12) comprises a battery; and/or
the power source (12) is arranged to provide a power of no more than 20W, no more than 10W, no more than 5W, and/or no more than 4W; and/or
the power source (12) comprises a transformer, preferably wherein the transformer is arranged to provide a high voltage; and/or
the power source (12) and/or a/the transformer is arranged to provide a voltage of at least 10kV.

7. The ion diffuser (10) of any preceding claim, comprising one or more of:
a placement structure (15) arranged to ensure a positioning of the substance in the cavity (11), preferably wherein the placement structure (15) comprises a magnet;
a distribution mechanism (16) arranged to distribute the ions, preferably wherein the distribution mechanism (16) comprises a fan;
a charged plate;
a substance identifier arranged to identify a characteristic of the substance and/or a/the capsule, preferably wherein:
the substance identifier identifies one or more of: a capacity and/or volume of the substance; a type of the substance; and an authorisation relating to the substance; and/or
the current provided to the substance depends on the characteristic, preferably wherein a duration and/or a magnitude of the current depends on the characteristic;
a filter arranged to selectively block the emission of matter from the ion diffuser; and
a processor and/or a user interface, preferably wherein the user interface is arranged to display a/the characteristic of the substance.

8. The ion diffuser (10) of any preceding claim, wherein the substance comprises one or more of:
a metal;
a halogen;
a compound, preferably a compound comprising a metal and a halogen;
a liquid acrylic polymer; and
one or more of: platinum, silver, gold, magnesium, and potassium.

9. A capsule (20) for use with an ion diffuser (10), the capsule (20) comprising:
a substance (21), wherein the substance comprises an ionic gel, the ionic gel comprising an ionic liquid that is held by an inorganic or polymer matrix;
wherein the capsule (20) is arranged to receive a current from the ion diffuser (10), the current causing the emission of ions from the substance;
preferably, wherein the capsule (20) is arranged to be refillable.

10. The capsule (20) of claim 9, wherein the capsule (20) comprises one or more of:
a metal;
a halogen;
a compound, preferably a compound comprising a metal and a halogen;
a liquid acrylic polymer; and
one or more of: platinum, silver, gold, magnesium, and potassium.

11. The capsule (20) of claim 9 or 10, comprising one or more of:
one or more electrodes (22, 23);
a power source, preferably a rechargeable power source;
an identifier, preferably wherein:
the identifier comprises a communication interface, more preferably wherein the identifier comprises a radio frequency identifier (RFID) interface and/or a near field communication (NFC) interface; and/or
the identifier is arranged to identify a characteristic of the capsule (20) and/or a substance in the capsule (20).

12. The capsule (20) of any of claims 9 to 11, wherein the capsule (20) comprises a plurality of substance sections (21-1, 21-2) separated by a gap (24), preferably wherein:
each of the substance sections (21-1, 21-2) comprises an ionic gel; and/or
at least two of the substance sections (21-1, 21-2) are positioned adjacent to an electrode (22, 23) of the capsule (20), preferably wherein at least one substance section is positioned adjacent to an anode (22) and at least one substance section is positioned adjacent to a cathode (23); and/or
a first substance section (22-1) is positioned adjacent to an anode (22) of the capsule (20) and a second substance section (21-2) is positioned adjacent to a cathode (23) of the capsule (20).

13. A method of operating an ion diffuser (10) for generating ions, the method comprising:
providing (41) a substance to a cavity (11) of the ion diffuser (10) wherein the substance comprises an ionic gel, the ionic gel comprising an ionic liquid that is held by an inorganic or polymer matrix; and
providing (42) a current to the substance to cause the emission of ions from the substance.

14. A method of manufacturing a capsule (20) for use in an ion diffuser (10), the capsule (20) comprising a substance, the substance being selected such that the receipt of a current from the ion diffuser causes the emission of ions from the substance;
wherein the substance comprises an ionic gel, the ionic gel comprising an ionic liquid that is held by an inorganic or polymer matrix.

## Patentansprüche

1. Ionendiffusor (10) zum Erzeugen von Ionen, wobei der Diffusor (10) umfasst:
einen Hohlraum (11), der dazu eingerichtet ist, eine Substanz aufzunehmen; wobei die Substanz sich in dem Hohlraum befindet; und
eine Energiequelle (12), die dazu eingerichtet ist, einen Strom an die Substanz bereitzustellen, um die Emission von Ionen von der Substanz zu bewirken;
wobei die Substanz ein ionisches Gel umfasst, wobei das ionische Gel eine ionische Flüssigkeit umfasst, die von einer anorganischen oder Polymermatrix gehalten wird.

2. Ionendiffusor (10) nach Anspruch 1, wobei der Ionendiffusor (10) dazu eingerichtet ist, positive Ionen und/oder sowohl positive als auch negative Ionen zu erzeugen.

3. Ionendiffusor (10) nach einem vorhergehenden Anspruch, wobei die Substanz ein Material und/oder ein Element mit einer Ordnungszahl von mehr als 8 umfasst.

4. Ionendiffusor (10) nach einem vorhergehenden Anspruch, wobei:
der Ionendiffusor (10) dazu eingerichtet ist, eine Vielzahl von unterschiedlichen Typen von Substanzen aufzunehmen; und/oder
der Ionendiffusor (10) eine Vielzahl von Hohlräumen umfasst, die dazu eingerichtet sind, eine Vielzahl von Substanzen aufzunehmen, wobei der Ionendiffusor (10) dazu eingerichtet ist, einen Strom selektiv an eine oder mehrere der Substanzen bereitzustellen, vorzugsweise auf der Basis einer Benutzereingabe; und/oder
der Ionendiffusor (10) dazu eingerichtet ist, einen Typ der oder jeder Substanz zu identifizieren.

5. Ionendiffusor (10) nach einem vorhergehenden Anspruch, der dazu eingerichtet ist, eine Kapsel (20) aufzunehmen, die die Substanz (21) umfasst, vorzugsweise wobei:
der Hohlraum (11) dazu eingerichtet ist, die Kapsel (20) aufzunehmen; und/oder
der Ionendiffusor (10) dazu eingerichtet ist, eine Vielzahl von Kapseln aufzunehmen, vorzugsweise wobei die Vielzahl von Kapseln unterschiedliche Substanzen umfasst;
mehr bevorzugt wobei:
der Hohlraum (11) dazu geeignet ist, eine Kapsel (20) aufzunehmen, die eine oder mehrere Elektroden (22, 23) umfasst, noch mehr bevorzugt wobei der Hohlraum (11) dazu eingerichtet ist, eine Kapsel (20) aufzunehmen, die eine Anode (22) und/oder eine Kathode (23) umfasst; und/oder
der Ionendiffusor (10) dazu eingerichtet ist, einen Typ der oder jeder Kapsel (20) zu identifizieren.

6. Ionendiffusor (10) nach einem vorhergehenden Anspruch, wobei:
der Ionendiffusor (10) eine oder mehrere Elektroden (13, 14) umfasst, die mit der Energiequelle (12) verbunden sind, vorzugsweise eine Anode und/oder eine Kathode; und/oder
die Energiequelle (12) dazu eingerichtet ist, einen Strom an eine Elektrode (22, 23) einer/der Kapsel (20) bereitzustellen und/oder wobei die Energiequelle (12) dazu eingerichtet ist, einen Funken an die Substanz bereitzustellen; und/oder
die Energiequelle (12) eine Batterie umfasst; und/oder
die Energiequelle (12) dazu eingerichtet ist, eine Energie von nicht mehr als 20 W, nicht mehr als 10 W, nicht mehr als 5 W und/oder nicht mehr als 4 W bereitzustellen; und/oder
die Energiequelle (12) einen Transformator umfasst, vorzugsweise wobei der Transformator dazu eingerichtet ist, eine hohe Spannung bereitzustellen; und/oder
die Energiequelle (12) und/oder ein/der Transformator dazu eingerichtet sind, eine Spannung von mindestens 10 kV bereitzustellen.

7. Ionendiffusor (10) nach einem vorhergehenden Anspruch, umfassend eine bzw. einen oder mehrere von:
einer Platzierungsstruktur (15), die dazu eingerichtet ist, eine Positionierung der Substanz in dem Hohlraum (11) sicherzustellen, vorzugsweise wobei die Platzierungsstruktur (15) einen Magneten umfasst;
einen Verteilungsmechanismus (16), der dazu eingerichtet ist, die Ionen zu verteilen, vorzugsweise wobei der Verteilungsmechanismus (16) ein Gebläse umfasst;
eine geladene Platte;
eine Substanzkennung, die dazu eingerichtet ist, ein Charakteristikum der Substanz und/oder einer/der Kapsel zu identifizieren, vorzugsweise wobei:
die Substanzkennung eines oder einen oder eine oder mehrere der folgenden identifiziert: ein Fassungsvermögen und/oder ein Volumen der Substanz; einen Typ der Substanz und eine Autorisierung in Bezug auf die Substanz; und/oder
der Strom, der an die Substanz bereitgestellt wird, von der Charakteristik abhängt, vorzugsweise wobei eine Dauer und/oder eine Größenordnung des Stroms von der Charakteristik abhängt;
einen Filter, der dazu eingerichtet ist, die Emission von Materie von dem Ionendiffusor selektiv zu blockieren; und
einen Prozessor und/oder eine Benutzerschnittstelle, vorzugsweise wobei die Benutzerschnittstelle dazu eingerichtet ist, ein/das Charakteristikum der Substanz anzuzeigen.

8. Ionendiffusor (10) nach einem vorhergehenden Anspruch, wobei die Substanz eines bzw. eine oder mehrere der folgenden umfasst:
ein Metall;
ein Halogen;
eine Verbindung, vorzugsweise eine Verbindung, die ein Metall oder ein Halogen umfasst;
ein flüssiges Acrylpolymer und
eines oder mehrere von: Platin, Silber, Gold, Magnesium und Kalium.

9. Kapsel (20) zur Verwendung mit einem Ionendiffusor (10), wobei die Kapsel (20) umfasst:
eine Substanz (21), wobei die Substanz ein ionisches Gel umfasst, wobei das ionische Gel eine ionische Flüssigkeit umfasst, die von einer anorganischen oder Polymermatrix gehalten wird;
wobei die Kapsel (20) dazu eingerichtet ist, einen Strom von dem Ionendiffusor (10) zu empfangen, wobei der Strom die Emission von Ionen von der Substanz bewirkt;
vorzugsweise wobei die Kapsel (20) dazu eingerichtet ist, wiederbefüllbar zu sein.

10. Kapsel (20) nach Anspruch 9, wobei die Kapsel (20) eines bzw. eine oder mehrere der folgenden umfasst:
ein Metall;
ein Halogen;
eine Verbindung, vorzugsweise eine Verbindung, die ein Metall oder ein Halogen umfasst;
ein flüssiges Acrylpolymer und
eines oder mehrere von: Platin, Silber, Gold, Magnesium und Kalium.

11. Kapsel (20) nach Anspruch 9 oder 10, umfassend eine oder mehrere von:
einer oder mehreren Elektroden (22, 23);
einer Energiequelle, vorzugsweise einer wiederaufladbaren Energiequelle;
einer Kennung, vorzugsweise wobei:
die Kennung eine Kommunikationsschnittstelle umfasst, mehr bevorzugt wobei die Kennung eine Radiofrequenzkennungsschnittstelle (RFID-Schnittstelle) und/oder eine Nahfeldkommunikationsschnittstelle (NFC-Schnittstelle) umfasst; und/oder
die Kennung dazu eingerichtet ist, ein Charakteristikum der Kapsel (20) und/oder einer Substanz in der Kapsel (20) zu identifizieren.

12. Kapsel (20) nach einem der Ansprüche 9 bis 11, wobei die Kapsel (20) eine Vielzahl von Substanzabschnitten (21-1, 21-2) umfasst, die durch eine Lücke (24) getrennt sind, vorzugsweise wobei:
jeder der Substanzabschnitte (21-1, 21-2) ein ionisches Gel umfasst; und/oder
mindestens zwei der Substanzabschnitte (21-1, 21-2) benachbart zu einer Elektrode (22, 23) der Kapsel (20) positioniert sind, vorzugsweise wobei mindestens ein Substanzabschnitt benachbart zu einer Anode (22) positioniert ist und mindestens ein Substanzabschnitt benachbart zu einer Kathode (23) positioniert ist; und/oder
ein erster Substanzabschnitt (22-1) benachbart zu einer Anode (22) der Kapsel (20) positioniert ist und ein zweiter Substanzabschnitt (21-2) benachbart zu einer Kathode (23) der Kapsel (20) positioniert ist.

13. Verfahren zum Betrieb eines Ionendiffusors (10) zum Erzeugen von Ionen, wobei das Verfahren umfasst:
Bereitstellen (41) einer Substanz an einen Hohlraum (11) des Ionendiffusors (10), wobei die Substanz ein ionisches Gel umfasst, wobei das ionische Gel eine ionische Flüssigkeit umfasst, die von einer anorganischen oder Polymermatrix gehalten wird; und
Bereitstellen (42) eines Stroms an die Substanz, um die Emission von Ionen von der Substanz zu bewirken.

14. Verfahren zur Herstellung einer Kapsel (20) zur Verwendung in einem Ionendiffusor (10), wobei die Kapsel (20) eine Substanz umfasst, wobei die Substanz derart ausgewählt ist, dass der Empfang eines Stroms von dem Ionendiffusor die Emission von Ionen von der Substanz bewirkt;
wobei die Substanz ein ionisches Gel umfasst, wobei das ionische Gel eine ionische Flüssigkeit umfasst, die von einer anorganischen oder Polymermatrix gehalten wird.

## Revendications

1. Diffuseur d'ions (10) conçu pour générer des ions, le diffuseur d'ions (10) comprenant :
une cavité (11) agencée pour recevoir une substance ; la substance se trouvant dans la cavité ; et
une source d'alimentation (12) agencée pour appliquer un courant à la substance afin de provoquer l'émission d'ions à partir de la substance ;
dans lequel la substance comprend un gel ionique, le gel ionique comprenant un liquide ionique qui est retenu par une matrice inorganique ou polymère.

2. Diffuseur d'ions (10) selon la revendication 1, le diffuseur d'ions (10) étant agencé pour générer des ions positifs et/ou à la fois des ions positifs et négatifs.

3. Diffuseur d'ions (10) selon l'une quelconque des revendications précédentes, dans lequel la substance comprend une matière et/ou un élément ayant un nombre atomique supérieur à 8.

4. Diffuseur d'ions (10) selon l'une quelconque des revendications précédentes,
le diffuseur d'ions (10) étant agencé pour recevoir une pluralité de différents types de substances ;
et/ou
le diffuseur d'ions (10) comprenant une pluralité de cavités agencées pour recevoir une pluralité de substances, le diffuseur d'ions (10) étant agencé pour appliquer sélectivement un courant à une ou plusieurs des substances, préférablement sur la base d'une entrée d'un utilisateur ; et/ou
le diffuseur d'ions (10) étant agencé pour identifier un type de la ou de chaque substance.

5. Diffuseur d'ions (10) selon l'une quelconque des revendications précédentes, lequel est agencé pour recevoir une capsule (20) comprenant la substance (21), préférablement dans lequel :
la cavité (11) est agencée pour recevoir la capsule (20) ; et/ou
le diffuseur d'ions (10) est agencé pour recevoir une pluralité de capsules, préférablement dans lequel les capsules constituant la pluralité comprennent différentes substances ;
plus préférablement dans lequel :
la cavité (11) est conçue pour recevoir une capsule (20) comprenant une ou plusieurs électrodes (22, 23), encore plus préférablement dans lequel la cavité (11) est agencée pour recevoir une capsule (20) comprenant une anode (22) et/ou une cathode (23) ; et/ou
le diffuseur d'ions (10) est agencé pour identifier un type de la ou de chaque capsule (20).

6. Diffuseur d'ions (10) selon l'une quelconque des revendications précédentes, dans lequel :
le diffuseur d'ions (10) comprend une ou plusieurs électrodes (13, 14) connectées à la source d'alimentation (12), préférablement une anode et/ou une cathode ; et/ou
la source d'alimentation (12) est agencée pour appliquer un courant à une électrode (22, 23) d'une/de la capsule (20) et/ou dans lequel la source d'alimentation (12) est agencée pour appliquer une étincelle à la substance ; et/ou
la source d'alimentation (12) comprend une batterie ; et/ou
la source d'alimentation (12) est agencée pour produire une puissance ne dépassant pas 20 W, ne dépassant pas 10 W, ne dépassant pas 5 W, et/ou ne dépassant pas 4 W ; et/ou
la source d'alimentation (12) comprend un transformateur, préférablement dans lequel le transformateur est agencé pour produire une haute tension ; et/ou
la source d'alimentation (12) et/ou un/le transformateur est agencé(e) pour produire une tension d'au moins 10 kV.

7. Diffuseur d'ions (10) selon l'une quelconque des revendications précédentes, comprenant un ou plusieurs de :
une structure de placement (15) agencée pour assurer un positionnement de la substance dans la cavité (11), préférablement dans lequel la structure de placement (15) comprend un aimant ;
un mécanisme de distribution (16) agencé pour distribuer les ions, préférablement dans lequel le mécanisme de distribution (16) comprend un ventilateur ;
une plaque chargée ;
un identificateur de substance agencé pour identifier une caractéristique de la substance et/ou d'une/de la capsule, préférablement dans lequel :
l'identificateur de substance identifie un ou plusieurs de : une capacité et/ou un volume de la substance ; un type de la substance ; et une autorisation relative à la substance ; et/ou
le courant appliqué à la substance dépend de la caractéristique, préférablement dans lequel une durée et/ou une grandeur du courant dépendent de la caractéristique ;
un filtre agencé pour bloquer sélectivement l'émission de matière à partir du diffuseur d'ions ; et
un processeur et/ou une interface utilisateur, préférablement dans lequel l'interface utilisateur est agencée pour afficher une/la caractéristique de la substance.

8. Diffuseur d'ions (10) selon l'une quelconque des revendications précédentes, dans lequel la substance comprend un ou plusieurs de :
un métal ;
un halogène ;
un composé, préférablement un composé comprenant un métal et un halogène ;
un polymère acrylique liquide ; et
un ou plusieurs du platine, de l'argent, de l'or, du magnésium et du potassium.

9. Capsule (20) destinée à une utilisation avec un diffuseur d'ions (10), la capsule (20) comprenant :
une substance (21), dans laquelle la substance comprend un gel ionique, le gel ionique comprenant un liquide ionique qui est retenu par une matrice inorganique ou polymère ;
la capsule (20) étant agencée pour recevoir un courant en provenance du diffuseur d'ions (10), le courant provoquant l'émission d'ions à partir de la substance ;
la capsule (20) étant préférablement agencée pour être rechargeable.

10. Capsule (20) selon la revendication 9, la capsule (20) comprenant un ou plusieurs de :
un métal ;
un halogène ;
un composé, préférablement un composé comprenant un métal et un halogène ;
un polymère acrylique liquide ; et
un ou plusieurs du platine, de l'argent, de l'or, du magnésium et du potassium.

11. Capsule (20) selon la revendication 9 ou 10, comprenant un ou plusieurs de :
une ou plusieurs électrodes (22, 23) ;
une source d'alimentation, préférablement une source d'alimentation rechargeable ;
un identificateur, préférablement dans laquelle :
l'identificateur comprend une interface de communication, plus préférablement dans laquelle l'identificateur comprend une interface d'identification par radiofréquence (RFID) et/ou une interface de communication en champ proche (NFC) ; et/ou
l'identificateur est agencé pour identifier une caractéristique de la capsule (20) et/ou d'une substance présente dans la capsule (20).

12. Capsule (20) selon l'une quelconque des revendications 9 à 11, la capsule (20) comprenant une pluralité de sections de substance (21-1, 21-2) séparées par un espace (24), préférablement dans laquelle :
chacune des sections de substance (21-1, 21-2) comprend un gel ionique ; et/ou
au moins deux des sections de substance (21-1, 21-2) sont positionnées de façon adjacente à une électrode (22, 23) de la capsule (20), préférablement dans laquelle au moins une section de substance est positionnée de façon adjacente à une anode (22) et au moins une section de substance est positionnée de façon adjacente à une cathode (23) ; et/ou
une première section de substance (22-1) est positionnée de façon adjacente à une anode (22) de la capsule (20) et une deuxième section de substance (21-2) est positionnée de façon adjacente à une cathode (23) de la capsule (20).

13. Procédé de fonctionnement d'un diffuseur d'ions (10) conçu pour générer des ions, le procédé comprenant :
l'apport (41) d'une substance à une cavité (11) du diffuseur d'ions (10), dans lequel la substance comprend un gel ionique, le gel ionique comprenant un liquide ionique qui est retenu par une matrice inorganique ou polymère ; et
l'application (42) d'un courant à la substance pour provoquer l'émission d'ions à partir de la substance.

14. Procédé de fabrication d'une capsule (20) destinée à une utilisation dans un diffuseur d'ions (10), la capsule (20) comprenant une substance, la substance étant sélectionnée de telle sorte que la réception d'un courant en provenance du diffuseur d'ions provoque l'émission d'ions à partir de la substance ;
dans lequel la substance comprend un gel ionique, le gel ionique comprenant un liquide ionique qui est retenu par une matrice inorganique ou polymère.
